(19) Europäisches Patentamt
European Patent Office
Office européen des brevets

(11) Veröffentlichungsnummer: **0 009 710**
**B1**

(12) # EUROPÄISCHE PATENTSCHRIFT

(45) Veröffentlichungstag der Patentschrift: **03.03.82**

(51) Int. Cl.³: **C 07 C 119/02,** A 01 N 35/10, A 01 N 37/18

(21) Anmeldenummer: **79103489.5**

(22) Anmeldetag: **17.09.79**

(54) **Alpha-Isocyano-cyclopropan-carbonsäureamide, Verfahren zu ihrer Herstellung und ihre Verwendung als Pflanzenwachstumsregulatoren.**

(30) Priorität: **29.09.78 DE 2842639**

(43) Veröffentlichungstag der Anmeldung: **16.04.80 Patentblatt 80/8**

(45) Bekanntmachung des Hinweises auf die Patenterteilung: **03.03.82 Patentblatt 82/9**

(84) Benannte Vertragsstaaten: **BE CH DE FR GB IT NL**

(56) Entgegenhaltungen:
**EP-A-0 002 014**
**DE-A-2 218 009**
**DE-A-2 405 819**
**US-A-4 098 600**
**CHEMISCHE BERICHTE, 109, 1976**
**Verlag Chemie GmbH, Seiten 482–487**
**Weinheim, DE.**
**I. HOPKE et al.: «Cycloaddition von «Isocyanketen» an Benzophenonanil und Thioimidsäureester zu β-Lactamen»**

(73) Patentinhaber: **BAYER AG, Zentralbereich Patente, Marken und Lizenzen, D-5090 Leverkusen 1, Bayerwerk (DE)**

(72) Erfinder: **Schröder, Rolf, Dr., Pahlkestrasse 17, D-5600 Wuppertal-1 (DE)**
Erfinder: **Lürssen, Klaus, Dr., August-Kierspel-Strasse 89, D-5070 Bergisch- Gladbach 2 (DE)**

**EP 0 009 710 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# α-Isocyano-cyclopropan-carbonsäureamide, Verfahren zu ihrer Herstellung und ihre Verwendung als Pflanzenwachstumsregulatoren

Die Erfindung betrifft neue α-Isocyano-cyclopropan-carbonsäureamide, ein Verfahren zu ihrer Herstellung sowie ihre Verwendung zur Regulierung des Pflanzenwachstums.

Es ist bereits bekannt geworden, dass (2-Chlorethyl)-trimethylammoniumchlorid pflanzenwuchsregulierende Eigenschaften aufweist (vgl. US-PS 3 156 554). Die Wirksamkeit dieses Stoffes ist jedoch, – vor allem bei niedrigen Aufwandmengen –, nicht immer ganz befriedigend.

Weiterhin ist bereits bekannt geworden, dass sich bestimmte Isocyano-acylamide, die sich von nicht-cyclischen aliphatischen Carbonsäuren ableiten, zur Regulierung des Pflanzenwachstums einsetzen lassen (vgl. US-PS 4 098 600). Auch ihre Wirkung lässt jedoch bei niedrigen Aufwandmengen zu wünschen übrig.

Es wurden nun neue α-Isocyano-cyclopropancarbonsäureamide der Formel

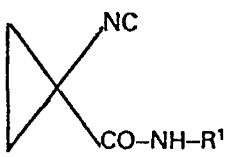

(I)

in welcher

$R^1$ für Wasserstoff, Alkyl mit 1 bis 18 Kohlenstoffatomen, Allyl oder Benzyl steht, gefunden.

Weiterhin wurde gefunden, dass man die α-Isocyanocyclopropan-carbonsäureamide der Formel (I) erhält, wenn man α-Isocyano-cyclopropan-carbonsäureester der Formel

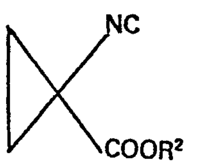

(II)

in welcher

$R^2$ für niederes Alkyl steht, mit Aminen der Formel

$R^1-NH_2$ (III)

in welcher

$R^1$ die oben angegebene Bedeutung hat, gegebenenfalls unter Verwendung eines Verdünnungsmittels umsetzt.

Ausserdem wurde gefunden, dass die α-Isocyano-cyclopropan-carbonsäureamide der Formel (I) sich durch starke pflanzenwuchsregulierende Eigenschaften auszeichnen.

Überraschenderweise zeigen die erfindungsgemässen α-Isocyano-cyclopropan-carbonsäureamide eine wesentlich höhere pflanzenwuchsregulierende Wirksamkeit als das aus dem Stand der Technik bekannte (2-Chlorethyl)-trimethyl-ammoniumchlorid, welches ein hochaktiver Wirkstoff gleicher Wirkungsart ist. Ausserdem besitzen die erfindungsgemässen Stoffe bessere pflanzenwuchsregulierende Eigenschaften als das aus der US-PS 4 098 600 bekannte N-methyl--isocyanoacetamid. Ferner übertreffen sie in ihrer Aktivität auch andere konstitutionell ähnliche Verbindungen, die den in der US-PS 4 098 600 beschriebenen Stoffen entsprechen.

Als Beispiele für erfindungsgemässe α-Isocyano-cyclopropan-carbonsäureamide der Formel (I) seien im einzelnen aufgeführt:

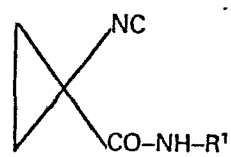

(I)

$R^1$ = $n-C_4H_9$
$i-C_3H_7$
$n-C_3H_7$
sek.-$C_4H_9$
$n-C_5H_{11}$
$n-C_6H_{13}$
$n-C_7Z_{15}$

Verwendet man beispielsweise α-Isocyano-cyclopropan-carbonsäuremethylester und n-Dodecylamin als Ausgangsstoffe, so kann der Reaktionsablauf des erfindungsgemässen Verfahrens durch das folgende Formelschema wiedergegeben werden:

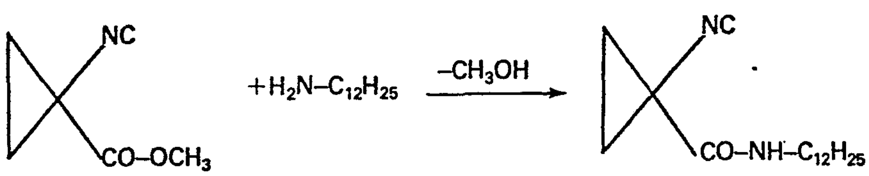


Die bei der Durchführung des erfindungsgemässen Verfahrens als Ausgangsstoffe benötigten α-Isocyano-cyclopropan-carbonsäureester sind durch die Formel (II) allgemein definiert. In dieser Formel steht $R^2$ vorzugsweise für geradkettiges oder verzweigtes Alkyl mit 1 bis 4 Kohlenstoffatomen, insbesondere für Methyl oder Ethyl.

Als Beispiele für α-Isocyano-cyclopropancarbonsäureester der Formel (II) seien genannt: α-Isocyano-cyclopropan-carbonsäuremethylester und -ethylester.

Die α-Isocyano-cyclopropan-carbonsäureester der Formel (II) sind bekannt oder können nach bekannten Verfahren hergestellt werden (verglei-

che Chem. Ber. 108 (1975), 1580–1592; Angew. Chem. 83 (1971), 357–358 und DE-OS 2 063 502).

Die bei dem erfindungsgemässen Verfahren weiterhin als Ausgangsstoffe zu verwendenden Amine sind durch die Formel (III) allgemein definiert.

Als Beispiele seien im einzelnen genannt:

Ammoniak, Methylamin, Ethylamin, Propylamin, iso-Propylamin, Butylamin, iso-Butylamin, Pentylamin, Hexylamin, Octylamin, Decylamin, Dodecylamin, Tetradecylamin, Hexadecylamin, Octadecylamin, Allylamin und Benzylamin.

Die Verbindungen der Formel (III) sind bekannt.

Das Verfahren zur Herstellung der erfindungsgemässen α-Isocyano-cyclopropan-carbonsäureamide wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether, wie Diethyl- und Dibutylether, Tetrahydrofuran und Dioxan, Ketone wie Aceton, Methylethyl-, Methylisopropyl- und Methylisobutylketon, Nitrile wie Acetonitril und Propionitril sowie Alkohole wie Methanol, Ethanol und Isopropanol.

Die Reaktionstemperaturen können bei der Durchführung des erfindungsgemässen Verfahrens innerhalb eines grösseren Bereiches variiert werden. Im allgemeinen arbeitet man bei Temperaturen zwischen 0 °C und 100 °C, vorzugsweise zwischen 10 °C und 80 °C.

Das erfindungsgemässe Verfahren wird im allgemeinen bei Normaldruck durchgeführt.

Zur Durchführung des erfindungsgemässen Verfahrens werden die Ausgangsstoffe im allgemeinen in äquimolaren Mengen eingesetzt. Ein Überschuss der einen oder anderen Reaktionskomponente bringt keine wesentlichen Vorteile. Die Umsetzung wird im allgemeinen in einem geeigneten Verdünnungsmittel durchgeführt und das Reaktionsgemisch wird mehrere Stunden bei der erforderlichen Temperatur gerührt. Dann wird das Reaktionsgemisch auf 0 bis 20 °C abgekühlt und vom ausgefallenen Produkt abgesaugt. Zur Charakterisierung dient der Schmelzpunkt. Soweit die Produkte nicht kristallin anfallen, werden sie durch Abdestillieren des Lösungsmittels isoliert. Zur Charakterisierung dient dann der Brechungsindex.

Die erfindungsgemässen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können z.B. zur Hemmung des vegetativen Pflanzenwachstums eingesetzt werden. Eine derartige Wuchshemmung ist unter anderen bei Gräsern von wirtschaftlichem Interesse, denn durch eine Dämpfung des Graswachstums kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag bezogen auf die Bodenfläche erzielt werden kann.

Ein weiterer Mechanismus der Ertragssteigerung mit Wuchshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z.B. mehr oder grössere Früchte zur Ausbildung kommen.

Ertragssteigerungen können in manchen Fällen auch durch einen Eingriff in den pflanzlichen Stoffwechsel erreicht werden, ohne dass sich Änderungen des vegetativen Wachstums bemerkbar machen. Wachtstumsregulatoren können ferner eine Veränderung der Zusammensetzung der Pflanzen bewirken, um so eine bessere Qualität der Ernteprodukte herbeizuführen. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Unter dem Einfluss von Wachstumsregulatoren kann der Blattbestand von Pflanzen so gesteuert werden, dass ein Entblättern der Pflanzen zu einem gewünschten Zeitpunkt erreicht wird. Eine derartige Entlaubung ist von Interesse, um eine mechanische Beerntung, z.B. bei Wein oder Baumwolle, zu erleichtern oder um die Transpiration zu einem Zeitpunkt herabzusetzen, an dem die Pflanze verpflanzt werden soll.

Durch Einsatz von Wachstumsregulatoren lässt sich der vorzeitige Fruchtfall verhindern. Es ist jedoch auch möglich, den Fruchtfall, – z.B. bei Obst –, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht bzw. eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Hilfe von Wachstumsregulatoren auch eine zeitliche Konzentrierung der Reife erzielt werden. Damit werden die Voraussetzungen dafür geschaffen, dass z.B. bei Tabak, Tomaten oder Kaffee, eine vollständige mechanische oder manuelle Beerntung in nur einem Arbeitsgang vorgenommen werden kann.

Durch Anwendung von Wachstumsregulatoren kann auch die Samen- oder Knospenruhe der Pflanzen, also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden.

Wachstumsregulatoren können auch eine Halophilie bei Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Einweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide; Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate , angewendet werden. Die Anwendung geschieht in üblicher Weise, z.B. durch Giessen, Verspritzen, Versprühen, Verstreuen, Verstäuben, Verschäumen, Begasen usw. Es ist ferner möglich, die Wirkstoffe nach dem Ultra-Low-Volume-Verfahren auszubringen, Pflanzen oder Pflanzenteile mit der Wirkstoffzubereitung oder dem Wirkstoff selbst zu bestreichen oder die Wirkstoffzubereitung oder den Wirkstoff selbst in den Boden zu injizieren. Es kann auch das Saatgut der Pflanze behandelt werden.

Die Wirkstoffkonzentrationen können in einem grösseren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, dass die Anwendung der Wachstumsregulatoren in einem bevorzugten Zeitraum vorgenommen wird, dessen genaue Abgrenzung sich nach dem klimatischen und vegetativen Gegebenheiten richtet.

Im folgenden wird die Aktivität der erfindungsgemässen α-Isocyano-cyclopropan-carbonsäureamide der Formel (I) als Pflanzenwachstumsregulatoren illustriert.

In dem Beispiel wird der nachstehend angegebene Stoff als Vergleichssubstanz eingesetzt

$$A = Cl{-}CH_2{-}CH_2{-}\overset{\oplus}{N}(CH_3)_3 \quad Cl^{\ominus}$$

(2-Chlorethyl)-trimethylammoniumchlorid

Verwendungsbeispiel

Stimulation der Ethylenbiosynthese bzw. Ethylenabspaltung

| | |
|---|---|
| Lösungsmittel: | 30 Gewichtsteile Dimethylformamid |
| Emulgator: | 1 Gewichtsteil Polyoxyethylen-Sorbitan-Monolaurat |

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Aus Sojabohnenblättern werden Blattstücke gleicher Grösse gestanzt. Diese werden zusammen mit 1 ml der jeweiligen Wirkstoffzubereitung bzw. Kontroll-Lösung in luftdicht verschliessbare Gefässe gegeben. Nach einer Stunde werden diese Gefässe verschlossen. Nach weiteren 24 Stunden wird das Ethylen, das sich in den Gefässen angesammelt hat, mit üblichen Nachweismethoden bestimmt. Die Ethylenentwicklung der mit Wirkstoffzubereitung behandelten Blattstücke wird mit der Ethylenentwicklung der Kontrollen verglichen.

Es bedeuten:

| | |
|---|---|
| 0 | keine Wirkung |
| + | schwache Stimulation der Ethylenbiosynthese |
| ++ | mittlere Stimulation der Ethylenbiosynthese |
| +++ | starke Stimulation der Ethylenbiosynthese |

Dieser Text ist in besonderem Masse geeignet, die wachstumsregulierenden Eigenschaften der erfindungsgemässen Verbindungen zu verdeutlichen.

Das Pflanzenhormon Ethylen greift in zahlreiche Prozesse bei der Entwicklung der Pflanzen ein. Eine Erhöhung der Ethylenmenge, wie sie mit den erfindungsgemässen Substanzen erzielt werden kann, erlaubt es, diese Prozesse zu steuern. Als Beispiele für die möglichen Wirkungen, für die ein besonderes kommerzielles Interesse besteht, seien hier genannt: Fruchtablösung, Reifebeschleunigung von Früchten und Blättern, Blühinduktion, Samenkeimung, Fruchtausdünnung, Stimulation des Latexflusses z.B. bei Hevea, Geschlechtsbeeinflussung und Wuchshemmung z.B. auch um das Lagern von Getreide zu verhindern.

Die Auswertung der Versuchsergebnisse zeigt, dass

– die erfindungsgemässen Verbindungen (2) und (5) eine geringe Stimulation der Ethylenbiosynthese bewirken,

– die erfindungsgemässen Verbindungen (1) und (4) eine mittlere Stimulation der Ethylenbiosynthese bewirken, und

– die erfindungsgemässe Verbindung (3) eine starke Stimulation der Ethylenbiosynthese bewirkt,

– während die bekannte Vergleichssubstanz (A) keine Stimulation der Ethylenbiosynthese bewirkt.

Herstellungsbeispiel

Beispiel 1

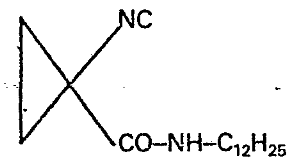

(1)

Eine Lösung von 50 mMol α-Isocyanocyclopropan-carbonsäure-ethylester und 50 mMol Dodecylamin in 100 ml Methanol wird 4 Stunden bei 50 °C gerührt. Danach wird abgekühlt, der ausgefallene Feststoff abgesaugt und mit einigen Millilitern Methanol nachgespült. Es verbleibt α-Isocyano-cyclopropan-carbonsäure-dodecylamid mit einem Schmelzpunkt von 44 °C. Ausbeute: 86% der Theorie

Analog Beispiel 1 werden folgende Verbindungen der Formel

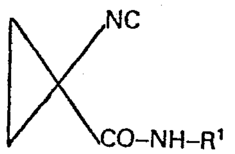

(I)

hergestellt:

| Beispiel Nr. | $R^1$ | Ausbeute (% der Theorie) | Schmelzpunkt (°C) |
|---|---|---|---|
| 2 | $-C_{14}H_{29}$ | 85 | 46 |
| 3 | $-C_{18}H_{37}$ | 83 | |
| 4 | H | 91 | 148 |
| 5 | $CH_3$ | 70 | 52 |

**Patentansprüche**

1) α-Isocyano-cyclopropan-carbonsäureamide der Formel

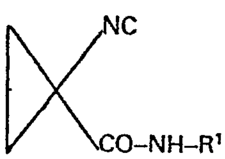

(I)

in welcher

$R^1$ für Wasserstoff, Alkyl mit 1 bis 18 Kohlenstoffatomen, Allyl oder Benzyl steht.

2) Verfahren zur Herstellung von α-Isocyanocyclopropan-carbonsäureamiden der Formel (I) gemäss Anspruch 1, dadurch gekennzeichnet, dass man α-Isocyanocyclopropan-carbonsäure-ester der Formel

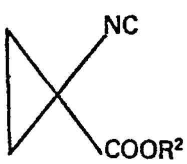

(II)

in welcher

$R^2$ für niederes Alkyl steht, mit Aminen der Formel

$R^1-NH_2$ (III)

in welcher

$R^1$ die oben angegebene Bedeutung hat, gegebenenfalls unter Verwendung eines Verdünnungsmittels umsetzt.

3) Mittel zur Regulierung des Pflanzenwachstums, gekennzeichnet durch einen Gehalt an mindestens einem α-Isocyanocyclopropan-carbonsäureamide der Formel (I) gemäss Anspruch 1.

4) Verfahren zur Regulierung des Pflanzenwachstums, dadurch gekennzeichnet, dass man α-Isocyanocyclopropan-carbonsäureamide der Formel (I) gemäss Anspruch 1 auf die Pflanzen oder ihren Lebensraum einwirken lässt.

5) Verwendung von α-Isocyanocyclopropan-carbonsäureamiden der Formel (I) gemäss Anspruch 1 zur Regulierung des Pflanzenwachstums.

6) Verfahren zur Herstellung von Pflanzenwachstumsregulierenden Mitteln, dadurch gekennzeichnet, dass man α-Isocyanocyclopropan-carbonsäureamide der Formel (I) gemäss Anspruch 1 mit Streckmitteln und/oder oberflächenaktiven Mitteln vermischt.

**Revendications**

1. Amides d'acide α-isocyano-cyclopropane-carboxylique de formule:

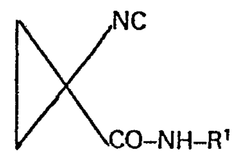

(I)

dans laquelle

$R^1$ est l'hydrogène, un reste alkyl ayant 1 à 18 atomes de carbone, allyle ou benzyle.

2. Procédé de production d'amides d'acide α-isocyano-cyclopropane-carboxylique de formule (I) suivant la revendication 1, caractérisé en ce qu'on fait réagir des esters d'acide α-isocyano-cyclopropane-carboxylique de formule:

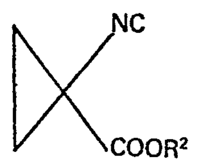

(II)

dans laquelle

$R^2$ est un reste alkyle inférieur, avec des amines de formule

$R^1-NH_2$ (III)

dans laquelle

$R^1$ a la définition indiquée ci-dessus, éventuellement en utilisant un diluant.

3. Composition destinée à la régulation de croissance des plantes, caractérisée par une teneur en au moins un amide d'acide α'-isocyanocyclopropane-carboxylique de formule (I) suivant la revendication 1.

4. Procédé de régulation de la croissance des végétaux, caractérisé en ce qu'on fait agir des amides d'acide α-isocyanocyclopropane-carboxylique de formule (I) suivant la revendication 1 sur les plantes ou sur leur milieu.

5. Utilisation d'amides d'acide α-isocyanocyclopropane-carboxylique de formule (I) suivant la revendication 1 pour la régulation de la croissance des plantes.

6. Procédé de préparation de compositions de régulation de la croissance des plantes, caractérisé en ce qu'on mélange des amides d'acide α-isocyanocyclopropane-carboxylique de formule (I) suivant la revendication 1 avec des diluants et/ou des agents tensio-actifs.

**Claims**

1. α-Isocyano-cyclopropane-carboxylic acid amides of the formula

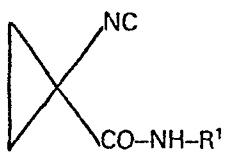

(I)

in which

$R^1$ represents hydrogen, alkyl with 1 to 18 carbon atoms, allyl or benzyl.

2. Process for the preparation of α-isocyano-cyclopropane-carboxylic acid amides of the formula (I) according to Claim 1, characterised in that α-isocyano-cyclopropane-carboxylic acid esters of the formula

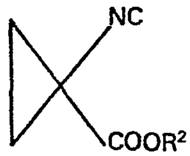

(II)

in which

$R^2$ represents lower alkyl are reacted with amines of the formula

$$R^1\text{–}NH_2 \qquad (III)$$

in which

$R^1$ has the meaning indicated above,

if appropriate using a diluent.

3. Agents for regulating plant growth, characterised in that they contain at least one α-isocyano-cyclopropane-carboxylic acid amide of the formula (I) according to Claim 1.

4. Process for regulating plant growth, characterised in that α-isocyano-cyclopropane-carboxylic acid amides of the formula (I) according to Claim 1 are allowed to act on the plants or their environment.

5. Use of α-isocyano-cyclopropane-carboxylic acid amides of the formula (I) according to Claim 1 for regulating plant growth.

6. Process for the preparation of plant growth-regulating agents, characterised in that α-isocyano-cyclopropane-carboxylic acid amides of the formula (I) according to Claim 1 are mixed with extenders and/or surface-active agents.